(19) Europäisches Patentamt
European Patent Office
Office européen des brevets

(11) Veröffentlichungsnummer: **0 063 684**
**A1**

(12) # EUROPÄISCHE PATENTANMELDUNG

(21) Anmeldenummer: **82101815.7**

(51) Int. Cl.³: **C 07 C 149/18**, G 02 B 1/04

(22) Anmeldetag: **08.03.82**

(30) Priorität: **27.04.81 CH 2730/81**
**18.01.82 CH 272/82**

(43) Veröffentlichungstag der Anmeldung: **03.11.82**
**Patentblatt 82/44**

(84) Benannte Vertragsstaaten: **AT BE CH DE FR IT LI NL**

(71) Anmelder: **F. HOFFMANN-LA ROCHE & CO. Aktiengesellschaft, CH-4002 Basel (CH)**

(72) Erfinder: **Petrzilka, Martin, Dr., Violaweg 74, CH-4303 Kaiseraugst (CH)**
Erfinder: **Pracht, Inge, Rössligasse 41, CH-4125 Riehen (CH)**
Erfinder: **Ruf, Urs, Dr., Im Bruckacker 23, CH-4105 Biel-Benken (CH)**

(74) Vertreter: **Cottong, Norbert A. et al, Grenzacherstrasse 124 Postfach 3255, CH-4002 Basel (CH)**

(54) **Immersionsöl.**

(57) Es werden Thioverbindungen der Formel

$$R^1O-(CH_2)_{n_1}-S-(CH_2)_{m_1}-OR^2 \qquad I$$

worin $R^1$ Wasserstoff, Benzoyl oder eine Gruppe $-(CH_2)_{n_2}-OR^3$ und $R^2$ Wasserstoff oder eine Gruppe $-(CH_2)_{m_2}-OR^4$ bezeichnen, $R^3$ Wasserstoff oder eine Gruppe $-(CH_2)_{n_3}-OH$ und $R^4$ Wasserstoff oder eine Gruppe $-(CH_2)_{m_3}-OH$ darstellen, die Indices $n_1$, $m_1$, $n_2$, $m_2$, $n_3$ und $m_3$ ganze Zahlen von 2 bis 6 bedeuten und die Summen $n_1 + m_1$ bzw. $n_2 + m_2$ bzw. $n_3 + m_3$ höchstens 8 betragen,
und Derivate hiervon, worin eine oder mehrere der Methylgruppen durch Alkylreste mit 1 bis 4 Kohlenstoffatomen substituiert sind, zur Verwendung für Immersionsöle, ein neues Immersionsöl sowie dessen Verwendung in der Optik, insbesondere in der Fluoreszenzmikroskopie beschrieben.

Das neue Immersionsöl ist gut UV-durchlässig, praktisch fluoreszenzfrei und nicht aggressiv gegenüber Glas und Kunststoffmaterialien.



ACTORUM AG

RAN 6200/6

# Immersionsöl

Die vorliegende Erfindung betrifft aliphatische Thioverbindungen, nämlich Thioglykole und Monoester hiervon, zur Verwendung für Immersionsöle, ein neues Immersionsöl sowie dessen Verwendung in der Optik, insbesondere in der Fluoreszenzmikroskopie.

Die Bezeichnung "Immersionsöl" ist ein in der Mikroskopie geläufiger Fachausdruck und bedeutet nicht Oele im sonst üblichen Sinne, sondern umfasst allgemein Immersionsmittel, welche für eine Immersionsoptik geeignet sind. Die Bezeichnung "Thioglykole" soll zum Ausdruck bringen, dass die betreffenden Verbindungen zwei Hydroxygruppen und eine Thioäthergruppe aufweisen.

Die Oelimmersionsoptik, bei der das Immersionsöl einen Teil des optischen Systems bildet und deshalb genau auf dieses abgestimmt sein muss, ist in der modernen Mikroskopie weit verbreitet. Das Immersionsöl füllt hierbei den Zwischenraum zwischen Objektiv und Deckglas bzw. zu untersuchendem Präparat vollständig aus und ermöglicht auf diese Weise bei der mikroskopischen Untersuchung eine höhere Auflösung und Helligkeit sowie eine wesentlich geringere sphärische Aberration als die trockene Optik (Brechungs-

Zim/27.1.82

index $n_D$ von Luft 1,000) oder die Wasserimmersionsoptik (Brechungsindex $n_D$ von Wasser 1,332). Der Brechungsindex $n_D$ des Immersionsöles sollte zu diesem Zweck zumindest dem Glas der Objektivfrontlinse angepasst sein, d.h. etwa 1,500 bis etwa 1,525 (bei 23°C) betragen. Nach Uebereinkunft der Mikroskophersteller und entsprechenden Festlegungen des Deutschen Institutes für Normen soll ein Immersionsöl folgende Werte aufweisen: $n_D$ (23°C) = 1,515, $n_e$ (23°C) = 1,518 $\pm$ 0,0004 und Dispersion $\nu_e$ = 44 $\pm$ 5. Ferner sollte das Immersionsöl gut UV-durchlässig und möglichst fluoreszenzfrei sein und weder Glas, Kunststoff-Objektträger noch die meisten zu untersuchenden Proben angreifen. Das Immersionsöl soll zudem farblos, möglichst geruchlos und nicht hygroskopisch sein, eine Viskosität besitzen, die eine bequeme Handhabung ermöglicht, auf den Benützer keinen schädigenden Einfluss haben und eine konstante Zusammensetzung aufweisen, d.h. es darf insbesondere keine optische Veränderung durch den Einfluss von Licht, Luft, Temperatur und dergleichen eintreten.

Die bisher bekannten Immersionsöle bestanden meistens aus Mischungen von Mineralölen, Paraffinölen und/oder Polyisobutylen mit Verbindungen von hohem Brechungsindex, wie beispielsweise polychlorierte Biphenyle (PCB) oder hydrierte Terphenyle (US-Patent No. 3,929,667). PCB-Verbindungen sind bekannt als toxisch und umweltgefährdend und werden deshalb nur noch verwendet, wo es wegen ihrer günstigen Fluoreszenzeigenschaften unbedingt erforderlich ist. Die anderen Verbindungen, wie z.B. die hydrierten Terphenyle, weisen hingegen den Nachteil einer mässigen bis geringen, aber doch störenden Fluoreszenz im UV-Licht und einer schlechteren Durchlässigkeit auf. PCB-Verbindungen greifen zudem Kunststoffmaterialien, wie beispielsweise Kunststoff-Objektträger, an.

Aufgabe der vorliegenden Erfindung war daher die Bereitstellung eines Immersionsöles, welches den optischen Anforderungen genügt und zugleich die Nachteile der vorbekannten Immersionsöle nicht oder in geringerem Masse aufweist.

Ueberraschenderweise wurde nun gefunden, dass mit einem Immersionsöl auf der Basis aliphatischer Thioverbindungen alle gewünschten Eigenschaften erreicht werden können. Insbesondere hat sich gezeigt, dass aliphatische Thioverbindungen sehr gut UV-durchlässig, praktisch fluoreszenzfrei (UV- bis Blau-Anregung) und nicht aggressiv gegenüber Glas, Kunststoffmaterialien oder den am häufigsten zu untersuchenden Proben sind. Naturgemäss stehen diejenigen Thioverbindungen im Vordergrund des Interesses, welche in Bezug auf Geruch und Oekotoxikologie unbedenklich sind, namentlich 2,2'-Thiodiäthanol (Thiodiäthylenglykol), dessen Benzoesäure-monoester und mit diesen eng verwandte Verbindungen.

Gegenstand der vorliegenden Erfindung ist somit ein Immersionsöl für optische Zwecke enthaltend eine oder mehrere aliphatische Thioverbindungen der allgemeinen Formel

$$R^1O-(CH_2)_{n_1}-S-(CH_2)_{m_1}-OR^2 \qquad I$$

worin $R^1$ Wasserstoff, Benzoyl oder eine Gruppe $-(CH_2)_{n_2}-OR^3$ und $R^2$ Wasserstoff oder eine Gruppe $-(CH_2)_{m_2}-OR^4$ bezeichnen, $R^3$ Wasserstoff oder eine Gruppe $-(CH_2)_{n_3}-OH$ und $R^4$ Wasserstoff oder eine Gruppe $-(CH_2)_{m_3}-OH$ darstellen, die Indices $n_1$, $m_1$, $n_2$, $m_2$, $n_3$ und $m_3$ ganze Zahlen von 2 bis 6 bedeuten und die Summen $n_1+m_1$ bzw. $n_2+m_2$ bzw. $n_3+m_3$ höchstens 8 betragen,

oder Derivate hiervon, worin eine oder mehrere der Methylengruppen durch Alkylreste mit 1 bis 4 Kohlenstoffatomen substituiert sind.

Weiterhin betrifft die vorliegende Erfindung die Verwendung der oben definierten aliphatischen Thioverbindungen für Immersionsöle sowie die Verwendung des erfindungsgemässen Immersionsöles in der Optik, insbesondere in der Fluoreszenzmikroskopie.

Die beschriebenen Thioverbindungen sind bekannte oder Analoge bekannter Verbindungen und ihre Herstellung kann daher als dem Fachmann bekannt vorausgesetzt werden.

Die erfindungsgemässen Immersionsöle können wasserlöslich oder nicht wasserlöslich sein. Immersionsöle auf der Basis der oben definierten Thioverbindungen, worin $R^1$ Wasserstoff oder eine Gruppe $-(CH_2)_{n_2}-OR^3$ bezeichnet (d.h. aliphatische Thioglykole), sind wasserlöslich, und Immersionsöle auf der Basis der oben definierten Thioverbindungen, worin $R^1$ Benzoyl bezeichnet (d.h. Thioglykolmonoester), sind nicht wasserlöslich.

Wasserlösliche Immersionsöle haben den Vorteil, dass die Reinigung der Objektive und Objektivträger wesentlich erleichtert wird. Anderseits besteht bei wasserlöslichen Immersionsölen die Gefahr, dass Farbstoff aus nicht eingedeckten, gefärbten mikroskopischen Schnitten oder Ausstrichpräparaten (z.B. Blutausstrichen) herausgelöst wird oder nicht genügend fixierte Objekte abschwimmen. In solchen Fällen wird daher mit Vorteil ein nicht wasserlösliches Immersionsöl verwendet.

Der Brechungsindex $n_D$ des erfindungsgemässen Immersionsöles beträgt bei 23°C zweckmässig etwa 1,500 bis etwa 1,525 und vorzugswe[illegible] etwa 1,515.

Von den oben definierten Thioverbindungen sind diejenigen bevorzugt, worin $R^3$ oder $R^4$, vorzugsweise beide Reste, Wasserstoff bedeuten. Besonders bevorzugt sind diejenigen Verbindungen, worin $R^2$ und $R^3$ Wasserstoff bedeuten, und insbesondere diejenigen, worin $R^1$ Wasserstoff oder Benzoyl und $R^2$ Wasserstoff bedeuten. Die Summen $n_1 + m_1$ bzw. $n_2 + m_2$ bzw. $n_3 + m_3$ betragen vorzugsweise höchstens

6 (d.h., falls jeweils beide betroffenen Reste vorhanden sind, betragen die Summen 4 bis 6). Von den erfindungsgemäss verwendeten Thioverbindungen sind ferner diejenigen bevorzugt, welche höchstens 2 Alkylreste aufweisen. Bevorzugter Alkylrest ist die Methylgruppe. Weiterhin sind die nicht alkylsubstituierten Verbindungen, d.h. die Verbindungen der Formel I, gegenüber den alkylsubstituierten Thioverbindungen bevorzugt.

Ganz besonders bevorzugte Thioverbindungen sind somit diejenigen Verbindungen der Formel I, worin $R^1$ Wasserstoff oder Benzoyl bezeichnet, $R^2$ Wasserstoff bedeutet und die Summe $n_1 + m_1$ 4 bis 6 beträgt, sowie deren Methyl- und Dimethylderivate. Wichtigste Vertreter solcher Verbindungen sind 2,2'-Thiodiäthanol (Thiodiäthylenglykol) und 2-[(2-Hydroxyäthyl)thio]äthylbenzoat.

Als Beispiele besonders bevorzugter Thioglykole können folgende genannt werden:

$HOCH_2CH_2-S-CH_2CH_2OH$

$HOCH_2CH_2-S-CH_2CH_2CH_2OH$

$HOCH_2CH_2CH_2-S-CH_2CH_2CH_2OH$

$HOCH_2CH_2-S-CH_2CH(CH_3)OH$

$HOCH_2CH_2-S-CH_2C(CH_3)_2OH$

$C_6H_5COOCH_2CH_2-S-CH_2CH_2OH$

Der den im Handel erhältlichen Verbindungen oft anhaftende Geruch kann im allgemeinen durch übliche Reinigungsverfahren, z.B. Chromatographie und/oder Destillation, leicht beseitigt werden.

Die Brechungsindices $n_D$ der erfindungsgemäss verwendeten Thioverbindungen liegen innerhalb des für ein gutes Immersionsöl erforderlichen Bereiches von etwa 1,500 bis etwa 1,525 bei 23°C oder nahe an diesem Bereich. Je nach Anwendungszweck kann der Brechungsindex des Immersionsöles durch Zusatz geeigneter Verbindungen mit niedrigerem oder höherem Brechungsindex auf den gewünschten Wert optimiert werden. Derartige Zusätze sind grundsätzlich bekannt. Beispiele bevorzugter Zusätze zur Erhöhung des Brechungsindex sind 2-(Hydroxymethyl)thiophen ($n_D$ = 1,562) oder für wassermischbare Immersionsöle auch 2-Hydroxyäthyl-disulfid ($n_D$ = 1,565). Beispiele bevorzugter Zusätze zur Senkung des Brechungsindex sind für wassermischbare Immersionsöle aliphatische Glykole (z.B. Polyäthylenglykole und 2-Alkyl-1,3-propandiole), 1,3-Dimethoxy-2-propanol ($n_D$ = 1,418) und Alkanole mit 1 bis 10 Kohlenstoffatomen und für nicht wassermischbare Immersionsöle Lycopan ($n_D$ = 1,458), Isophytol ($n_D$ = 1,455), 1,2,3-Trimethoxypropan ($n_D$ = 1,402), 1,3-Dimethoxy-2-propanol ($n_D$ = 1,418), Phythylmethyläther ($n_D$ = 1,453) und Alkanole mit 11 bis 20 Kohlenstoffatomen. Besonders geeignet sind zur Senkung des Brechungsindex für nicht wassermischbare Immersionsöle Isophytol und für wassermischbare Immersionsöle aliphatische Glykole, z.B. die unter Handelsnamen wie Carbowax® und dergleichen bekannten Polyäthylenglykole verschiedener Polymerisationsgrade. Je nach gewünschter Viskosität und gewünschtem Temperaturbereich setzt man im allgemeinen Polyäthylenglykole unterschiedlicher Polymerisationsgrade ein (durchschnittliches Molekulargewicht zweckmässig zwischen etwa 200 und etwa 10'000).

Beispielsweise besitzt 2,2'-Thiodiäthanol (Thiodiäthylenglykol), welches die bevorzugteste Thioglykolverbindung darstellt, einen Brechungsindex von $n_D^{23}$ = 1,5203, welcher leicht, z.B. durch Zusatz von Polyäthylenglykol, erniedrigt werden kann.

Die Thioverbindungen können grundsätzlich in den erfindungsgemässen Immersionsölen in beliebiger Menge vorhanden sein. Der Anteil der gegebenenfalls vorhandenen Zusätze wird durch den Anwendungszweck und insbesondere durch den gewünschten Brechungsindex bestimmt. Im allgemeinen wird man jedoch bestrebt sein, einen möglichst

hohen Anteil an Thioverbindungen zu verwenden. Zweckmässigerweise beträgt daher der Anteil an Thioverbindungen, worin $R^1$ Wasserstoff oder eine Gruppe $-(CH_2)_{n_2}-OR^3$ bezeichnet, in den erfindungsgemässen, wasserlöslichen Immersionsölen mindestens etwa 50 Gew.-% und vorzugsweise mindestens etwa 70 Gew.-%. Die nicht wasserlöslichen Immersionsöle enthalten zweckmässig mindestens etwa 40 Gew.-% und vorzugsweise mindestens etwa 50 Gew.-% an Thioverbindungen, worin $R^1$ Benzoyl bedeutet. Die vorliegende Erfindung umfasst aber selbstverständlich auch Immersionsöle, welche nur Thioverbindungen enthalten.

Die Herstellung der aus 2 oder mehreren Komponenten bestehenden Immersionsöle und die Optimierung des Brechungsindex kann in an sich bekannter Weise erfolgen, z.B. durch Mischen der ungefähren Mengen der Komponenten bei erhöhter Temperatur, Abkühlen, Messen des Brechungsindex und, gegebenenfalls, Zugabe der entsprechenden Komponente (bzw. Komponenten), bis der gewünschte Brechungsindex erreicht ist.

Die oben erwähnte Fluoreszenzfreiheit ist so zu verstehen, dass die Fluoreszenz des Immersionsöles in einer horizontalen Quarzküvette von 0,2 mm Schichtdicke bei der quantitativen Messung im Fluoreszenzmikroskop von Wasser nicht signifikant verschieden ist. Je nach Art der Zusätze und Reinheit der Komponenten zeigen die erfindungsgemässen Immersionsöle keine oder eine sehr geringe Fluoreszenz.

Die erfindungsgemässen Immersionsöle können in verschiedenen Bereichen der Optik Verwendung finden. Beispielsweise können sie aufgrund der hohen Durchlässigkeit anstelle von Immersionsglycerin und in der Absorptionsphotometrie verwendet werden. Weitere verwandte Anwendungsgebiete sind: als optische Eichsubstanz, flüssiges Trägermedium, optisches Kupplungsmedium (Glasfaseroptik) und als transparente Untersuchungsflüssigkeit zur Feststellung von Materialunterschieden (z.B. Prüfung von Kontaktlinsen oder

in der Elektronikindustrie). Bevorzugtes Anwendungsgebiet ist jedoch die Mikroskopie und insbesondere die Fluoreszenzmikroskopie.

Die Erfindung betrifft ferner alle neuen Verbindungen, Mischungen, Verfahren, Verwendungen und Vorrichtungen wie hierin beschrieben.

Die vorliegende Erfindung wird durch die folgenden Beispiele weiter veranschaulicht. Die Eigenfluoreszenz wurde in Quarzküvetten von 0,2 mm Schichtdicke mit einem ZEISS-Mikroskop-Fluorometer für die drei Anregungen UV, Violett und Blau gemessen. Die relative Fluoreszenz wurde durch Vergleich mit Wasser (0%) und einem Glasstandard (100%) ermittelt.

## Beispiel 1

Ca. 90 Gewichtsteile 2,2'-Thiodiäthanol (Thiodiäthylenglykol, Reinheit mindestens 99%) und ca. 10 Gewichtsteile Carbowax®1000 (Polyäthylenglykol mit durchschnittlichem Molekulargewicht ca. 1000; reinste Qualität) werden unter Erwärmen gemischt, bis eine klare, homogene Flüssigkeit erhalten wird. Nach dem Abkühlen auf 23°C wird der Brechungsindex $n_D$ kontrolliert und gegebenenfalls durch weitere Zugabe einer der beiden Komponenten genau eingestellt.

Das erhaltene Immersionsöl ist farblos, fast geruchlos, wassermischbar, nicht aggressiv gegen Glas und Kunststoff-Objektträger und hat folgende technische Eigenschaften:

| | |
|---|---|
| Brechungsindices bei 23°C | $n_F$ (486,10 nm) = 1,523<br>$n_e$ (546,07 nm) = 1,518<br>$n_D$ (589,26 nm) = 1,515<br>$n_C$ (656,30 nm) = 1,512 |
| Temperaturabhängigkeit des Brechungsindex (18-30°C) | $\Delta n/\Delta T$ = 0,00035/°C |
| Dispersion (Abbé) | $\nu_e$ = 45 |
| Fluoreszenz (UV-, Violett- und Blauanregung | von destilliertem Wasser im Mikroskop nicht zu unterscheiden |
| Dichte bei 23°C | 1,18 g/ml |
| Viskosität bei 23°C | 74,0 mPa·s |
| Trübungspunkt | ca. -20°C |
| Spektrale Durchlässigkeit $\tau_i$ (0,2 mm Schichtdicke) | 50% bei ca. 260 nm, > 95% bei 300 nm |

## Beispiel 2

In analoger Weise zu Beispiel 1 werden ca. 60 Gewichtsteile 2-[(2-Hydroxyäthyl)thio]äthylbenzoat und ca. 40 Gewichtsteile Isophytol gemischt und dann der Brechungsindex $n_D$ genau eingestellt.

Das erhaltene Immersionsöl ist farblos, fast geruchlos, nicht wassermischbar, nicht hygroskopisch, nicht aggressiv gegen Glas, Kunststoff-Objektträger und Blutausstriche und hat folgende weiteren Eigenschaften:

| | |
|---|---|
| Brechungsindices bei 23°C | $n_F$ (486,10 nm) = 1,525<br>$n_e$ (546,07 nm) = 1,518<br>$n_D$ (589,26 nm) = 1,515<br>$n_C$ (656,30 nm) = 1,511 |
| Temperaturabhängigkeit des Brechungsindex (18-30°C) | $\Delta n/\Delta T = 0{,}00038/°C$ |
| Dispersion (Abbé) | $\nu_e = 37$ |
| Dichte bei 23°C | 1,05 g/ml |
| Viskosität bei 23°C | 72 mPa· s |
| Spektrale Durchlässigkeit $\tau_i$ (0,2 nm Schichtdicke) | 50% bei ca. 300 nm, > 95% bei 330 nm |
| Relative Fluoreszenz | 9% für UV-Anregung, 3% für Violett-Anregung, 3% für Blau-Anregung (Standardabweichung 1,9%) |

## Beispiel 3

In analoger Weise zu Beispiel 1 werden folgende Mischungen hergestellt:

a) Ca. 90 Gewichtsteile 2,2'-Thiodiäthanol (Thiodiäthylenglykol) und ca. 10 Gewichtsteile Polyäthylenglykol 4000 (Merck); farblos, wassermischbar; $n_D^{23} = 1{,}515$; Fluoreszenz für UV-, Violett- und Blau-Anregung von destilliertem Wasser im Mikroskop nicht zu unterscheiden.

b) Ca. 70 Gewichtsteile 2,2'-Thiodiäthanol (Thiodiäthylenglykol) und ca. 30 Gewichtsteile 4-[(2-Hydroxyäthyl)thio]-2-methyl-2-butanol; farblos, wassermischbar; $n_D^{23}$ = 1,515; Fluoreszenz für Violett- und Blau-Anregung von destilliertem Wasser im Mikroskop nicht zu unterscheiden; relative Fluoreszenz für UV-Anregung 5,7% (Standardabweichung 1,9%).

## Beispiel 4

2,55 g Lithiumaluminiumhydrid werden unter Argonbegasung in 100 ml absolutem Tetrahydrofuran aufgeschlämmt und mit einer warmen Lösung von 12,0 g 3,3'-Thiodipropionsäure in 100 ml absolutem Tetrahydrofuran versetzt. Nach beendeter Zugabe wird das heterogene Reaktionsgemisch noch während 22 Stunden zum Rückfluss erhitzt. Nach dem Abkühlen wird das Reaktionsgemisch mit 12,0 ml einer gesättigten Kaliumcarbonat-Lösung versetzt, filtriert und eingeengt. Niederdruckchromatographie (0,4 bar) des resultierenden, farblosen Oels an Kieselgel mit Essigester und anschliessende Kugelrohrdestillation (150-170°C/0,15-0,09 mm Hg) ergibt 2,0 g 3,3'-Thiodi-1-propanol als farbloses, zähflüssiges Oel; $n_D^{23}$ = 1,5088; relative Fluoreszenz: 20% für UV-Anregung, 2% für Violett-Anregung, 5% für Blau-Anregung.

## Beispiel 5

a) Unter Argonbegasung wird ein Gemisch von 14,0 ml Mercaptoäthanol und 0,15 ml einer 40%-igen Lösung von Triton B in Methanol vorgelegt und unter Eiskühlung während 40 Minuten tropfenweise mit 36,6 ml Acrylsäuremethylester versetzt (die Innentemperatur soll 25°C nicht übersteigen). Nach beendeter Zugabe wird das Reaktionsgemisch noch 19 Stunden bei Raumtemperatur gerührt, dann überschüssiger Acrylsäuremethylester abdestilliert und das zurückbleibende Oel destilliert. Im Hauptlauf (Sdp. 157-161°C/12 mm Hg) werden 22,0 g (67%) 3-[(2-Hydroxyäthyl)thio]propionsäuremethylester als farblose Flüssigkeit erhalten.

b) 1,15 g Lithiumaluminiumhydrid werden unter Argonbegasung in 50 ml absolutem Tetrahydrofuran aufgeschlämmt und innert 15 Minuten mit einer Lösung von 5,0 g 3-[(2-Hydroxyäthyl)thio]propionsäuremethylester in 50 ml absolutem Tetrahydrofuran versetzt. Nach beendeter Zugabe wird das heterogene Reaktionsgemisch noch 3 Stunden zum Rückfluss erhitzt. Nach dem Abkühlen wird das Reaktionsgemisch mit ca. 5,0 ml gesättigter Kaliumcarbonat-Lösung versetzt, filtriert und das Filtrat eingeengt. Kugelrohrdestillation (150°C/0,25-0,15 mm Hg) des Rückstandes (4,18 g) ergibt 3,52 g (86%) 3-[(2-Hydroxyäthyl)thio]-1-propanol als farbloses, unangenehm riechendes Oel; $n_D^{23}$ = 1,5139. Zur Beseitigung des Geruches wird dieses Material durch Niederdruckchromatographie (0,5 bar) an Kieselgel mit Essigester und anschliessende Kugelrohrdestillation (150°C/0,27-0,20 mm Hg) zusätzlich gereinigt. Es resultieren 3,07 g (75%) 3-[(2-Hydroxyäthyl)thio]-1-propanol als viskoses, geruchloses Oel; $n_D^{23}$ = 1,5139; relative Fluoreszenz: 13% für UV-Anregung, 4% für Violett-Anregung, 4% für Blau-Anregung.

## Beispiel 6

Unter Argonbegasung werden 100 ml einer 1N Lösung von Methylmagnesiumbromid in Diäthyläther vorgelegt und tropfenweise mit einer Lösung von 5,0 g 3-[(2-Hydroxyäthyl)thio]propionsäuremethylester (hergestellt nach Beispiel 5) in 25 ml Diäthyläther so versetzt, dass ein leichter Rückfluss entsteht. Nach beendeter Zugabe wird das Reaktionsgemisch noch 16 Stunden bei Raumtemperatur gerührt. Anschliessend wird das überschüssige Grignardreagens durch vorsichtige Zugabe gesättigter Ammoniumchlorid-Lösung zerstört und das Reaktionsgemisch mit dreimal je 100 ml Methylenchlorid extrahiert. Die organischen Phasen werden über Magnesiumsulfat getrocknet und eingeengt. Niederdruckchromatographie (0,4 bar) des Rückstandes an Kieselgel mit Essigester und anschliessende Kugelrohrdestillation (150°C/0,23-0,18 mmHg) ergibt 2,25 g (46%) 4-[(2-Hydroxyäthyl)thio]-2-methyl-2-butanol als zähflüssiges, farbloses Oel; $n_D^{23}$ = 1,5027.

Beispiel 7

Unter Argonbegasung wird eine Lösung von 8,5 ml 2,2'-Thiodiäthanol (Thiodiäthylenglykol) in 100 ml Pyridin bei -5°C vorgelegt und innert 10 Minuten tropfenweise mit 9,5 ml Benzoylchlorid versetzt. Dabei erwärmt sich das Reaktionsgemisch auf ca. +8°C und gegen Ende der Zugabe beginnen weisse Kristalle auszufallen. Anschliessend wird das Reaktionsgemisch noch 36 Stunden bei Raumtemperatur gerührt und dann der grösste Teil des Pyridins am Rotationsverdampfer abdestilliert. Der semikristalline Rückstand wird auf 100 ml eiskalte 2N Salzsäure gegeben und dreimal mit je 100 ml Diäthyläther extrahiert. Die organischen Phasen werden zweimal mit je 100 ml Wasser und einmal mit 100 ml gesättigter Kochsalzlösung gewaschen, über Magnesiumsulfat getrocknet und eingeengt. Niederdruckchromatographie (0,5 bar) des Rückstandes (ca. 14 g) an Kieselgel unter Verwendung von Toluol/Essigester 3:1 als Laufmittel ergibt der Reihe nach 1,5 g (5,5%) an kristallinem Dibenzoat und 11 g (59%) 2-[(2-Hydroxyäthyl)thio]äthylbenzoat als farbloses, viskoses Oel. Nach Kugelrohrdestillation (160-170°C/0,20-0,16 mm Hg) des letzteren werden 9,7 g (52,4%) 2-[(2-Hydroxyäthyl)thio]äthylbenzoat in 99,4%-iger Reinheit erhalten; $n_D^{23}$ = 1,5566.

Patentansprüche

1. Immersionsöl enthaltend eine oder mehrere aliphatische Thioverbindungen der allgemeinen Formel

$$R^1O-(CH_2)_{n_1}-S-(CH_2)_{m_1}-OR^2 \qquad I$$

worin $R^1$ Wasserstoff, Benzoyl oder eine Gruppe $-(CH_2)_{n_2}-OR^3$ und $R^2$ Wasserstoff oder eine Gruppe $-(CH_2)_{m_2}-OR^4$ bezeichnen, $R^3$ Wasserstoff oder eine Gruppe $-(CH_2)_{n_3}-OH$ und $R^4$ Wasserstoff oder eine Gruppe $-(CH_2)_{m_3}-OH$ darstellen, die Indices $n_1$, $m_1$, $n_2$, $m_2$, $n_3$ und $m_3$ ganze Zahlen von 2 bis 6 bedeuten und die Summen $n_1+m_1$ bzw. $n_2+m_2$ bzw. $n_3+m_3$ höchstens 8 betragen,

oder Derivate hiervon, worin eine oder mehrere der Methylengruppen durch Alkylreste mit 1 bis 4 Kohlenstoffatomen substituiert sind.

2. Wasserlösliches Immersionsöl nach Anspruch 1 enthaltend eine oder mehrere der in Anspruch 1 definierten Verbindungen, worin $R^1$ Wasserstoff oder eine Gruppe $-(CH_2)_{n_2}-OR^3$ bezeichnet.

3. Nicht wasserlösliches Immersionsöl nach Anspruch 1 enthaltend eine oder mehrere der in Anspruch 1 definierten Verbindungen, worin $R^1$ Benzoyl bezeichnet.

4. Immersionsöl nach einem der Ansprüche 1 bis 3, dadurch gekennzeichnet, dass der Brechungsindex $n_D$ bei 23°C etwa 1,500 bis etwa 1,525 beträgt.

5. Immersionsöl nach Anspruch 4, dadurch gekennzeichnet, dass der Brechungsindex $n_D$ bei 23°C etwa 1,515 beträgt.

6. Immersionsöle nach einem der Ansprüche 1 bis 5,

dadurch gekennzeichnet, dass $R^3$ und $R^4$ Wasserstoff bedeuten.

7. Immersionsöle nach einem der Ansprüche 1 bis 6, dadurch gekennzeichnet, dass $R^1$ Wasserstoff, Benzoyl oder eine Gruppe $-(CH_2)_{n_2}-OR^3$ und $R^2$ und $R^3$ Wasserstoff bedeuten.

8. Immersionsöle nach Anspruch 7, dadurch gekennzeichnet, dass $R^1$ Wasserstoff oder Benzoyl und $R^2$ Wasserstoff bedeuten.

9. Immersionsöle nach einem der Ansprüche 1 bis 8, dadurch gekennzeichnet, dass die Summen $n_1 + m_1$ bzw. $n_2 + m_2$ bzw. $n_3 + m_3$ in Formel I höchstens 6 betragen.

10. Immersionsöle nach einem der Ansprüche 1 bis 9, dadurch gekennzeichnet, dass die in Anspruch 1 definierte Thioverbindung höchstens 2 Alkylreste aufweist.

11. Immersionsöle nach einem der Ansprüche 1 bis 10, dadurch gekennzeichnet, dass in der in Anspruch 1 definierten Thioverbindung gegebenenfalls vorhandene Alkylreste Methylgruppen sind.

12. Immersionsöle nach einem der Ansprüche 1 bis 10 enthaltend eine oder mehrere Thioverbindungen der in Anspruch 1 definierten Formel I.

13. Immersionsöl nach einem der Ansprüche 1 bis 11 enthaltend eine oder mehrere Thioverbindungen der in Anspruch 1 definierten Formel I, worin $R^1$ Wasserstoff oder Benzoyl bezeichnet, $R^2$ Wasserstoff bedeutet und die Summe $n_1 + m_1$ 4 bis 6 beträgt, und/oder deren Methyl- oder Dimethylderivate.

14. Immersionsöl nach Anspruch 13 enthaltend 2,2'-Thiodiäthanol oder 2-[(2-Hydroxyäthyl)thio]äthylbenzoat.

15. Wasserlösliches Immersionsöl nach Anspruch 14 bestehend aus 2,2'-Thiodiäthanol und Polyäthylenglykol.

16. Nicht wasserlösliches Immersionsöl nach Anspruch 14 bestehend aus 2-[(2-Hydroxyäthyl)thio]äthylbenzoat und Isophytol.

17. Verwendung der in Anspruch 1 definierten Thioverbindungen für Immersionsöle.

18. Verwendung eines Immersionsöles gemäss einem der Ansprüche 1 bis 16 in der Optik, insbesondere in der Fluoreszenzmikroskopie.

***

Europäisches Patentamt

**EUROPÄISCHER RECHERCHENBERICHT**

0063684

Nummer der Anmeldung

EP 82101815.7

| EINSCHLÄGIGE DOKUMENTE | | | KLASSIFIKATION DER ANMELDUNG (Int. Cl. 3) |
|---|---|---|---|
| Kategorie | Kennzeichnung des Dokuments mit Angabe, soweit erforderlich, der maßgeblichen Teile | betrifft Anspruch | |
| X | DE - C - 436 434 (I.G. FARBENINDUSTRIE AKTIENGESELLSCHAFT)<br>-- | 1,2,4, 7-13 | C 07 C 149/18<br>G 02 B 1/04 |
| X | DE - C - 362 445 (FARBWERKE MEISTER LUCIUS & BRÜNING)<br>---- | 1,2,4, 7-10, 12-14 | |

**RECHERCHIERTE SACHGEBIETE (Int. Cl. 3)**

G 02 B

C 07 C 149/00

C 09 K

**KATEGORIE DER GENANNTEN DOKUMENTE**

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| WIEN | 08-07-1982 | TENGLER |